# EUROPÄISCHE PATENTANMELDUNG

(11) **EP 1 891 965 A1**
(43) Veröffentlichungstag der Anmeldung: **27.02.2008**
(21) Anmeldenummer: 06076585.6
(22) Anmeldetag: 16.08.2006
(51) Int. Cl.: A61K 38/16, A61K 38/45, A61K 39/00, A61K 48/00, C12Q 1/48, C12Q 1/68

(54) **Hip-Kinase für Fertilitätskontrolle**

(71) Anmelder: Bayer Schering Pharma Aktiengesellschaft, 13353 Berlin (DE)
(72) Erfinder: Die Erfindernennung liegt noch nicht vor

(57) **Zusammenfassung**

Die vorliegende Erfindung betrifft die Verwendung einer pharmazeutischen Zusammensetzung, die als aktive Komponente mindestens eine HipK4-Nukleinsäure, ein HipK4-Polypeptid, Modulatoren der HipK4, HipK4-Antikörper, eine HipK4-Antisense und eine HipK4-RNAi eunthält zur Diagnose und Behandlung von Fertilitätsstörungen und zur Kontrazeption. Die Erifndung betrifft weiterhin die Verwendung dieser pharmazeutischen Zusammensetzung zur Herstellung eines Medikaments für die Behandlung von Fertilitätsstörungen.

SEQUENCE LISTING

<110> Schering AG

<120> Hip-Kinase für Fertilitätskontrolle

<130> 53441

<160> 4

<170> PatentIn version 3.1

<210> 1
<211> 2451
<212> DNA
<213> homo sapiens

<400> 1
agagccactc aacagcgctg gaacccattc ggtggggcct ggggcccctc atcccaagcc 60
aggagggttt ctggggaggg gtgcagcccc tggcagactg acagtgtggc ctgggggttt 120
gggggtgcca gggaagcagg ggccaacctc ataggaggag acacgagtgc ggttctcttt 180
cccccactgg ggggcctgct gtgtcagcag ccaggcggga ggcctgggcg gcagagccag 240
tggtacaggg gcctgggcag ggcggtgtct ggcagcagcg gcaccatgtc caccatccag 300
tcggagactg actgctacga catcatcgag gtcttgggca aggggacctt cggggaggta 360
gccaagggct ggcggcggag cacgggcgag atggtggcca tcaagatcct caagaatgac 420
gcctaccgca accgcatcat caagaacgag ctgaagctgc tgcactgcat gcgaggccta 480
gaccctgaag aggcccacgt catccgcttc cttgagttct tccatgacgc cctcaagttc 540
tacctggtct ttgagctgct ggagcaaaac cttttcgagt tccagaagga gaacaacttc 600
gcgcccctcc ccgcccgcca catccgtaca gtcaccctgc aggtgctcac agccctggcc 660
cggctcaagg agctggctat catccacgct gatctcaagc ctgagaacat catgctggtg 720
gaccagaccc gctgcccctt cagggtcaag gtgattgact tcggatccgc cagcattttc 780
agcgaggtgc gctacgtgaa ggagccatac atccagtcgc gcttctaccg ggcccctgag 840
atcctgctgg ggctgccctt ctgcgagaag gtggacgtgt ggtccctggg ctgcgtcatg 900
gctgagctgc acctgggctg gcctctctac cccggcaaca acgagtacga ccaggtgcgc 960

tacatctgcg aaacccaggg cctgcccaag ccacacctgt tgcacgccgc ctgcaaggcc 1020
caccacttct tcaagcgcaa cccccaccct gacgctgcca acccctggca gctcaagtcc 1080
tcggctgact acctggccga gacgaaggtg cgcccattgg agcgccgcaa gtatatgctc 1140
aagtcgttgg accagattga gacagtgaat ggtggcagtg tggccagtcg gctaaccttc 1200
cctgaccggg aggcgctggc ggagcacgcc gacctcaaga gcatggtgga gctgatcaag 1260
cgcatgctga cctgggagtc acacgaacgc atcagcccca gtgctgccct gcgccacccc 1320
ttcgtgtcca tgcagcagct gcgcagtgcc cacgagacca cccactacta ccagctctcg 1380
ctgcgcagct accgcctctc gctgcaagtg gaggggaagc cccccacgcc cgtcgtggcc 1440
gcagaagatg ggacccccta ctactgtctg gctgaggaga aggaggctgc gggtatgggc 1500
agtgtggccg gcagcagccc cttcttccga gaggagaagg caccaggtat gcaaagagcc 1560
atcgaccagc tggatgacct gagtctgcag gaggctgggc atgggctgtg gggtgagacc 1620
tgcaccaatg cggtctccga catgatggtc cccctcaagg cagccatcac tggccaccat 1680
gtgcccgact cgggccctga gcccatcctg gccttctaca gcagccgcct ggcaggccgc 1740
cacaaggccc gcaagccacc tgcgggttcc aagtccgact ccaacttcag caacctcatt 1800
cggctgagcc aggtctcgcc tgaggatgac aggccctgcc ggggcagcag ctgggaggaa 1860
ggagagcatc tcggggcctc tgctgagcca ctggccatcc tgcagcgaga tgaggatggg 1920
cccaacattg acaacatgac catggaagct gagaggccag accctgagct cttcgacccc 1980
agcagctgtc ctggagaatg gctgagtgag ccagactgca ccctggagag cgtcaggggc 2040
ccacgggctc aggggctccc accccgccgc tcccaccagc atggtccacc ccggggggcc 2100
accagcttcc tccagcatgt caccgggcac cactgatggt gattccaccc ctgcccatca 2160
ctgggggctg cgctagctgg gctggcattc cctcccaacc tgaactgctc ctcagagcca 2220
tctcctgaac ccacaaatta ttcttacaga aagatagtta tccagaaatt ctcattcccc 2280
gtctgcggtg cggtgcgtgc ctgcacacct ctcctaaaca cagcagggct ttggagtctg 2340
gcccatgctc cttggccaga aggacagcag gaaagggggc tgcaccccgc tggccctgcg 2400
ctcgcccttg gccctgctgc ctctgtctat ttcaatatag aactgttcag c 2451

<210> 2

<211> 616

<212> PRT

<213> Homo sapiens


<400> 2

Met Ser Thr Ile Gln Ser Glu Thr Asp Cys Tyr Asp Ile Ile Glu Val
1               5                   10                  15

Leu Gly Lys Gly Thr Phe Gly Glu Val Ala Lys Gly Trp Arg Arg Ser
            20                  25                  30

Thr Gly Glu Met Val Ala Ile Lys Ile Leu Lys Asn Asp Ala Tyr Arg
        35                  40                  45

Asn Arg Ile Ile Lys Asn Glu Leu Lys Leu Leu His Cys Met Arg Gly
    50                  55                  60

Leu Asp Pro Glu Glu Ala His Val Ile Arg Phe Leu Glu Phe Phe His
65                  70                  75                  80

Asp Ala Leu Lys Phe Tyr Leu Val Phe Glu Leu Leu Glu Gln Asn Leu
                85                  90                  95

Phe Glu Phe Gln Lys Glu Asn Asn Phe Ala Pro Leu Pro Ala Arg His
            100                 105                 110

Ile Arg Thr Val Thr Leu Gln Val Leu Thr Ala Leu Ala Arg Leu Lys
        115                 120                 125

Glu Leu Ala Ile Ile His Ala Asp Leu Lys Pro Glu Asn Ile Met Leu
    130                 135                 140

Val Asp Gln Thr Arg Cys Pro Phe Arg Val Lys Val Ile Asp Phe Gly
145                 150                 155                 160

Ser Ala Ser Ile Phe Ser Glu Val Arg Tyr Val Lys Glu Pro Tyr Ile
                165                 170                 175

Gln Ser Arg Phe Tyr Arg Ala Pro Glu Ile Leu Leu Gly Leu Pro Phe
            180                 185                 190

Cys Glu Lys Val Asp Val Trp Ser Leu Gly Cys Val Met Ala Glu Leu
        195                 200                 205

His Leu Gly Trp Pro Leu Tyr Pro Gly Asn Asn Glu Tyr Asp Gln Val
    210                 215                 220

Arg Tyr Ile Cys Glu Thr Gln Gly Leu Pro Lys Pro His Leu Leu His
225                 230                 235                 240

Ala Ala Cys Lys Ala His His Phe Phe Lys Arg Asn Pro His Pro Asp
                245                 250                 255

Ala Ala Asn Pro Trp Gln Leu Lys Ser Ser Ala Asp Tyr Leu Ala Glu
            260                 265                 270

Thr Lys Val Arg Pro Leu Glu Arg Arg Lys Tyr Met Leu Lys Ser Leu
        275                 280                 285

Asp Gln Ile Glu Thr Val Asn Gly Gly Ser Val Ala Ser Arg Leu Thr
    290                 295                 300

Phe Pro Asp Arg Glu Ala Leu Ala Glu His Ala Asp Leu Lys Ser Met
305                 310                 315                 320

Val Glu Leu Ile Lys Arg Met Leu Thr Trp Glu Ser His Glu Arg Ile
                325                 330                 335

Ser Pro Ser Ala Ala Leu Arg His Pro Phe Val Ser Met Gln Gln Leu
            340                 345                 350

Arg Ser Ala His Glu Thr Thr His Tyr Tyr Gln Leu Ser Leu Arg Ser
        355                 360                 365

Tyr Arg Leu Ser Leu Gln Val Glu Gly Lys Pro Pro Thr Pro Val Val
    370                 375                 380

Ala Ala Glu Asp Gly Thr Pro Tyr Tyr Cys Leu Ala Glu Glu Lys Glu
385                 390                 395                 400

Ala Ala Gly Met Gly Ser Val Ala Gly Ser Ser Pro Phe Phe Arg Glu
                405                 410                 415

Glu Lys Ala Pro Gly Met Gln Arg Ala Ile Asp Gln Leu Asp Asp Leu
            420                 425                 430

Ser Leu Gln Glu Ala Gly His Gly Leu Trp Gly Glu Thr Cys Thr Asn
        435                 440                 445

Ala Val Ser Asp Met Met Val Pro Leu Lys Ala Ala Ile Thr Gly His
    450                 455                 460

His Val Pro Asp Ser Gly Pro Glu Pro Ile Leu Ala Phe Tyr Ser Ser
465                 470                 475                 480

Arg Leu Ala Gly Arg His Lys Ala Arg Lys Pro Pro Ala Gly Ser Lys
                485                 490                 495

Ser Asp Ser Asn Phe Ser Asn Leu Ile Arg Leu Ser Gln Val Ser Pro
            500                 505                 510

Glu Asp Asp Arg Pro Cys Arg Gly Ser Ser Trp Glu Glu Gly Glu His
        515                 520                 525

Leu Gly Ala Ser Ala Glu Pro Leu Ala Ile Leu Gln Arg Asp Glu Asp
    530                 535                 540

Gly Pro Asn Ile Asp Asn Met Thr Met Glu Ala Glu Arg Pro Asp Pro
545                 550                 555                 560

Glu Leu Phe Asp Pro Ser Ser Cys Pro Gly Glu Trp Leu Ser Glu Pro
                565                 570                 575

Asp Cys Thr Leu Glu Ser Val Arg Gly Pro Arg Ala Gln Gly Leu Pro
            580                 585                 590

Pro Arg Arg Ser His Gln His Gly Pro Pro Arg Gly Ala Thr Ser Phe
        595                 600                 605

Leu Gln His Val Thr Gly His His
    610                 615

## Beschreibung

Die vorliegende Erfindung betrifft das Enzym Hip-Kinase 4, das im engen Zusammenhang mit der männlichen Infertilität steht. Es ist demgemäß zur Diagnose und Behandlung männlicher Infertilität sowie als Mittel zur Schaffung von Kontrazeptiva geeignet.

Derzeit sind etwa 15 % aller Paare in Deutschland ungewollt kinderlos, ihr Anteil nimmt ständig zu. Die Ursachen für die Unfruchtbarkeit liegen zu gleichen Teilen bei Mann und Frau. Während die Ursachen bei der Frau weitgehend erforscht und diagnostizierbar sind, können beim Mann nur in etwa 30 % der Fälle organische Ursachen festgestellt werden. Etwa ein Drittel der verbleibenden Fälle lassen sich auf Oligospermie unbekannten Ursprungs zurückführen, während die Ursachen bei den restlichen Fällen nach dem bisherigen Wissenstand noch unklar sind. Insbesondere bei der idiopathischen Infertilität, bei der trotz einer ausreichenden Anzahl von Spermien im Ejakulat keine Befruchtung der weiblichen Eizelle erfolgt (H.W.G. Baker, Male Reproductive Dysfunction, 341 ff, 1986), wird eine Störung der im Nebenhoden stattfindenden Spermienreifung vermutet.

Bei der Spermienreifung greifen eine Vielzahl von enzymatisch regulierten Prozessen ineinander. Es ist davon auszugehen, dass Störungen, die durch die Dysregulation eines Enzyms verursacht werden, zu Störungen der Spermienreifung und so zu befruchtungsunfähigen Spermien führen.

Die Aufgabe der vorliegenden Erfindung besteht in der Bereitstellung neuer Mittel, mit denen Störungen des Enzymstoffwechsels im Hoden des Menschen und insbesondere die männliche Infertilität diagnostiziert und therapiert werden können, und die zur Fertilitätskontrolle eingesetzt werden können.

Die Hip-Kinase 4 gehört zu einer neuen Familie von Enzymen, die in einem Hefe-Di-Hybrid Systems ("yeast two hybrid screen") mit Domänen anderer Proteine interagieren. Diese Proteine enthalten eine konservierte Proteinkinase-Domäne und eine mit anderen Proteinen interagierende Domäne, daher werden die Proteinkinasen als "homeodomain-interacting protein kinases (HipKs) bezeichnet. Bisher sind 4 Isoformen bekannt: HipK1, HipK2, HipK3 and HipK4 (Kim, Choi et al. 1998, J Biol Chem 273(40): 25875-9.). Diese Proteinkinasen gehören zu einer größeren Familie von Kinasen, die durch Tyrosinphosphorylierung reguliert werden und ihre Autophosphorylierung an Serin/Threonin- und an Tyrosinresten katalysieren, ihre Substrate jedoch nur an Serin/Threoninresten phosphorylieren (Zhang, Li et al. 2005, Genomics 85(1): 117-30). Die Hipk1, 2 und 3 konnten im Zellkern nachgewiesen werden, ebenso konnten einige Substrate identifiziert werden.

Über die HipK4 ist bisher weniger bekannt. Die Sequenz der HipK4 wurde erstmalig beansprucht in der Anmeldung WO04/087901 (Wyeth).

Bisher gab es jedoch keine funktionelle Information für die HipK4 (Kim, Choi et al. 1998, J Biol Chem 273(40): 25875-9; Moilanen, Karvonen et al. 1998, Mol Biol Cell 9(9): 2527-43; Rochat-Steiner, Becker et al. 2000, J Exp Med 192(8): 1165-74; Ecsedy, Michaelson et al. 2003, Mol Cell Biol 23(3): 950-60.; Zhang, Li et al. 2005; Zhang, Nottke et al. 2005, Proc Natl Acad Sci U S A 102(8): 2802-7).

In der vorliegenden Erfindung konnte nun erstmalig gezeigt werden, dass die HipK4 eine wichtige Rolle bei den Prozessen der Spermatogenese/Spermiogenese spielt.

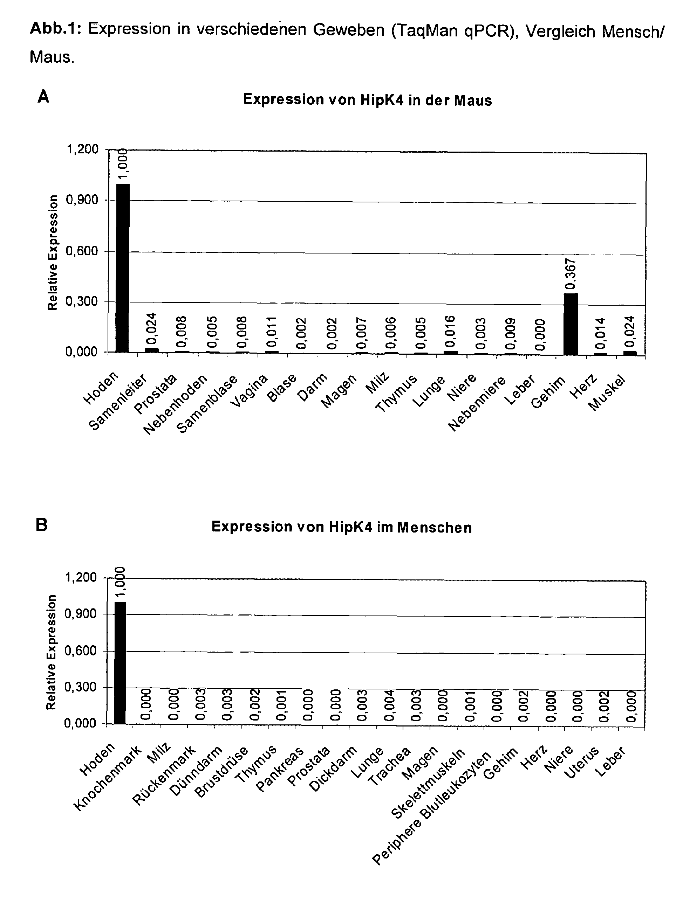

Es konnte gezeigt werden, dass die HipK4 vor allem im Hoden exprimiert wird. Die quantitative Gewebeverteilung der Hip-Kinase 4 wurde in Mäusen und Menschen mittels Q-RT-PCR bestimmt (Abb.1 A u. B). In Mäusen sowie im Menschen wird HipK4 vor allem im Hoden exprimiert. In der Maus konnte außerdem eine Expression im Gehirn nachgewiesen werden, die 37 % der Intensität des Expressionsniveaus des Hodens aufweist (Abb. 1 B).

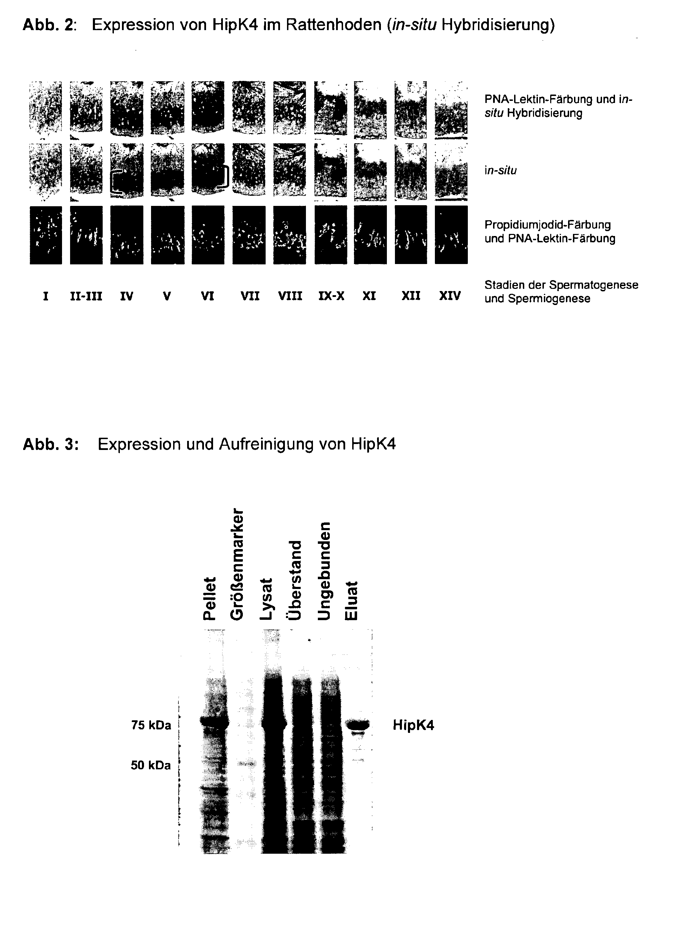

Die detaillierte Untersuchung der Expression von HipK4 mittels der in-situ Hybridisierung im Rattenhoden konnte nachweisen, dass HipK4 exklusiv in postmeiotischen runden Spermatiden exprimiert wird (Abb. 2). In Kombination mit einer PNA-Lektin (Aktrosomenfärbung) und Propidiumjodid-Färbung konnte die Expression von HipK4 während der Spermiogenese auf die Entwicklungsstufe 4-6 eingeschränkt werden (Abb. 2). Diese nahezu hodenspezifische, auf postmeiotische runde Spermatiden beschränkte HipK4-Expression, bestärkt die essentielle Rolle der Hip-Kinase 4 in der postmeiotischen Spermiogenese und unterstützt somit die Möglichkeit der Entwicklung eines Mittels zur Diagnose von Fertilitätsstörungen sowie zur Kontrazeption basierend auf der Inhibierung von HipK4.

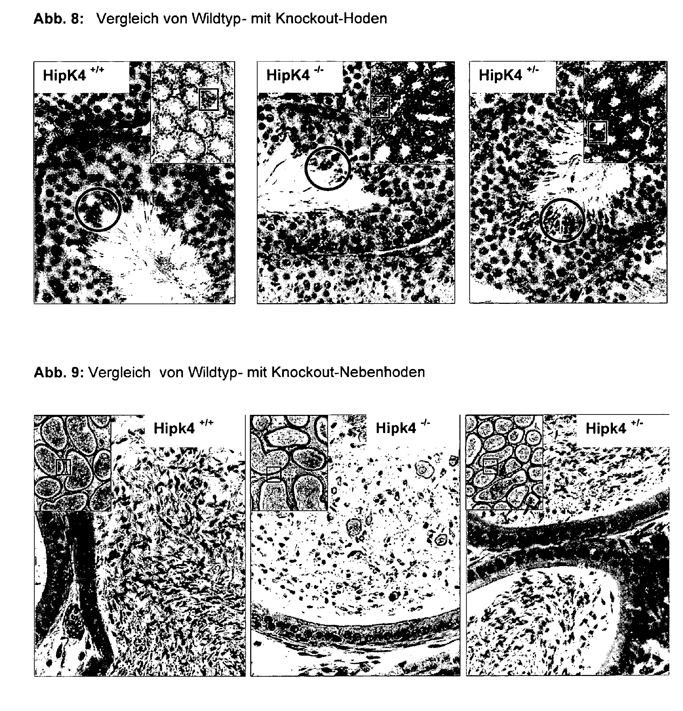

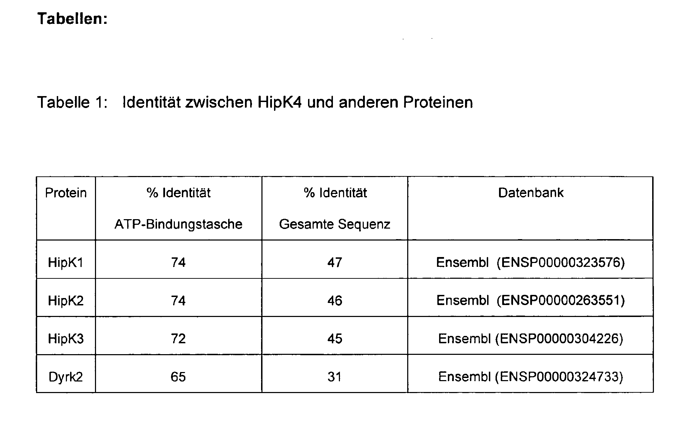

Mittels einer HipK4-knockout-Mauslinie konnte durch eine histologische Analyse von Hoden und Nebenhoden gezeigt werden, dass HipK4 eine essentielle Rolle während der Spermiogenese spielt (Abb. 8-9). Im Hoden führt der Mangel an HipK4 zu einer Beeinflussung der Spermiogenese, was sich in einer untypischen Anordnung der sich entwickelnden Spermatiden in den Hodenkanälchen zeigt (Abb. 8). Eine morphologische Veränderung der Spermatiden ist am stärksten in den Stadien kurz vor der Freisetzung der Spermien in den Lumen der Hodenkanälchen zu beobachten (Abb. 8 Kreis). Dabei weisen die Spermien der HipK4-defizienten Mauslinie nicht mehr die für dieses Entwicklungsstadium charakteristische längliche Form auf, sondern weisen eine veränderte Morphologie auf. Letztendlich führt dieser Spermiogenesedefekt zur Freisetzung einer drastisch verminderten Anzahl an Spermien im Hoden und somit zu einer Ansammlung dieser deformierten Spermien im Nebenhodenschwanz (Abb. 9).

Die Analyse der HipK4-defizienten Mauslinie konnte eindeutig die essentielle Funktion dieser Kinase in der Spermiogenese nachweisen. Somit ist anzunehmen, dass das Fehlen von HipK4 zur Infertilität führt. Modulatoren von HipK4 können folglich als neuartiges Arzneimittel zur Kontrazeption entwickelt und die Analyse der HipK4-Funktionalität zur Diagnose der Infertilität genutzt werden.

Daher löst die vorliegende Erfindung durch Bereitstellung einer neuen Therapiemethode, bei der das Enzym Hip-Kinase 4 und Modulatoren dieses Enzyms als Zielsubstanz für die Entwicklung neuer Arzneimittel dienen, die sowohl zur Diagnose der Infertilität als auch zur Fertilitätskontrolle geeignet sind, das vorliegende Problem.

Die vorliegende Erfindung betrifft die Verwendung einer pharmazeutischen Zusammensetzung, die als aktive Komponente mindestens eine Substanz, ausgewählt aus der Gruppe von einer HipK4-Nukleinsäure entsprechend Seq ID No 1, einem HipK4-Polypeptid entsprechend Seq ID No 2, Modulatoren der HipK4, Antikörpern, die gegen die HipK4 gerichtet sind, einer HipK4-Antisense sowie einer HipK4-RNAi, zur Diagnose und Behandlung von Fertilitätsstörungen, zur Kontrazeption und auch zur Herstellung eines Medikaments für die Behandlung von Fertilitätsstörungen.

HipK4-Nukleinsäuren umfassen sowohl einzel- oder doppelsträngige DNA, z.B. cDNA und RNA, z.B. mRNA, cRNA, pre-mRNA, bzw. Teile davon. Die DNA-und Proteinsequenzen sind in Seq ID No 1 und 4 dargestellt (Seq ID No 1: HipK4 Human: Nukleotidsequenz ID ENST00000291823 und Seq ID No 2: HipK4 Human Proteinsequenz ID ENSP00000291823). Die in Seq ID No 1 dargestellte Nukleinsäure kodiert für ein humanes HipK4 Protein.

Bevorzugt ist die Verwendung einer pharmazeutischen Zusammensetzung, in der die Nukleinsäure einen Protein kodierenden Abschnitt der in Seq ID No 1 dargestellten Nukleinsäuresequenz umfasst. Ein Protein kodierender Abschnitt der in Seq ID No 1 dargestellten Sequenz liegt im Bereich von 286 - 2136.

Die Nukleinsäure ist aus Säugern, z.B. humanen Zellen, oder aus einer cDNA-Bibliothek oder einer genomischen Bibliothek, die z.B. aus menschlichen Zellen gewonnen wird, erhältlich. Sie kann nach bekannten Techniken unter Verwendung kurzer Abschnitte der in Seq ID No 1 gezeigten Nukleinsäuresequenz als Hybridisierungssonden oder Amplifikationsprimer isoliert werden.

Weiterhin Gegenstand der Erfindung ist die oben genannte Verwendung einer pharmazeutischen Zusammensetzung, in der das Polypeptid die in Seq ID No 2 dargestellte Aminosäuresequenz umfasst.

Das Polypeptid kann ein rekombinantes Polypeptid, ein natürliches, isoliertes Polypeptid oder ein synthetisches Polypeptid sein.

Die mRNA des Polypeptids nach Seq ID No 2 wird in Hoden und in Keimzellen transkribiert.

Gegenstand der vorliegenden Erfindung ist die oben genannte Verwendung einer pharmazeutischen Zusammensetzung, die ein Polypeptidfragment entsprechend der Aminosäuresequenz gemäß Seq ID No 2 von Position 1 bis 616 enthält.

Das HipK4 Polypeptid oder Teilbereiche davon (Peptide) können zur Herstellung von Antikörpern verwendet werden. Zur Herstellung polyklonaler Antikörper können die Polypeptide oder Peptide z.B. an KLH (Keyhole Limpet Hemocyanin) gebunden werden und Tieren, z.B. Kaninchen, gespritzt werden. Sie können auch zur Herstellung monoklonaler Antikörper verwendet werden. Zur Antikörperherstellung kann ein HipK4-Polypeptid oder Peptid oder eine Mischung mehrerer HipK4-Peptide verwendet werden. Die Herstellung der Antikörper erfolgt dabei nach Standardverfahren, wie sie z.B. in Kohler, G. und Milstein, C., Nature 1975, 256, 495 - 497 und Nelson, P. N. et al., Mol. Pathol. 2000, 53, 111 - 117 beschrieben sind.

Gegenstand der Erfindung ist die Verwendung einer pharmazeutischen Zusammensetzung, die Antikörper beinhaltet, die gegen das HipK4-Polypeptid gerichtet sind, zur Diagnose von Fertilitätsstörungen.

Die Antikörper können zur Detektion der HipK4-Polypeptide verwendet werden. Dies kann z.B. durch Immunhistochemie erfolgen. Die Antikörper können auch in anderen Immuntests, wie z.B. einem ELISA (enzyme linked immunosorbent assay) oder in Radioimmunotests, eingesetzt werden. So kann die Konzentration an Polypeptid in Gewebe- oder Zellextrakten nachgewiesen werden.

Der Nachweis der Expression des Polypeptids kann auch über den Nachweis von mRNA in den Zellen erfolgen. Gegenstand der Erfindung ist daher auch die Verwendung einer Sonde mit Nukleinsäuresequenzen, die komplementär zu den Nukleinsäuresequenzen sind, die für das Polypeptid, das die in Seq ID No 2 dargestellte Aminosäuresequenz umfasst kodiert, zur Herstellung eines Reagenz zum Nachweis der Gegenwart von mRNA in Zellen. Eine Sonde ist ein kurzes RNA- oder DNA-Stück mit mindestens 14 Nukleotiden. Die erfindungsgemäßen Sonden können z.B. in einer Northern Blot Analyse verwendet werden. Diese Methode ist z.B. in Sambrook, J. et al., 1989, Cold Spring Harbor Laboratory Press, beschrieben. Andere Methoden zum Nachweis der RNA sind in situ Hybridisierung, RNAse Protection Assay oder PCR.

Die Erfindung betrifft außerdem die Verwendung einer pharmazeutischen Zusammensetzung, die Moleküle enthält, die gegen die Nukleinsäuresequenz gerichtet sind und die Expression des HipK4-Proteins unterdrücken können, wie z.B. Antisense-Moleküle und synthetische Moleküle, die die Stabilität oder Translation der HipK4-mRNA hemmen. Derartige Moleküle können gezielt zur Kontrazeption eingesetzt werden.

Gegenstand der vorliegenden Erfindung ist auch die Verwendung einer Zusammensetzung, die als aktive Komponente mindestens eine HipK4-Nukleinsäure, oder ein HipK4-Polypeptids oder ein HipK4-Antisense-Moleküls als Zielsubstanz zur Herstellung eines Mittels für die Behandlung von Krankheiten, die ursächlich mit dem HipK4-Gen und/oder Protein zusammenhängen, enthält.

Weiterhin betrifft die Erfindung Verfahren zur Diagnostik von Erkrankungen, deren Ursachen Mutationen des HipK4-Proteins beinhalten. Hierzu können DNA-Chips verwendet werden. Die Erfindung betrifft daher einen DNA-Chip, auf dem mindestens ein Oligonukleotid immobilisiert ist, das der vollständigen cDNA-Sequenz oder einer Teilsequenz bzw. komplementären Sequenz zu der in Seq ID No 1 beschriebenen, entspricht. Die Erfindung betrifft somit ferner die Verwendung eines erfindungsgemäßen DNA-Chips zur Diagnose von Fertilitätsstörungen u. a. im Hoden und Ovidukt.

DNA-Chips, auch als DNA-Mikroarrays bekannt, sind miniaturisierte Träger, meist aus Glas oder Silizium, auf deren Oberfläche DNA-Moleküle bekannter Sequenz in einem geordneten Raster in hoher Dichte immobilisiert werden. Die Oberflächen-gebundenen DNA-Moleküle werden mit komplementären, eventuell markierten Nukleinsäuren hybridisiert. Die Markierung kann ein Fluoreszenzfarbstoff sein.

Bei Oligonukleotid-Chips stellen die Oligonukleotide, die auf einem erfindungsgemäßen DNA-Chip gebunden sein können, Teilsequenzen der Genprodukte (mRNA bzw. daraus abgeleitete cDNA) dar. Es können ein oder mehrere Oligonukleotide pro Gen auf dem DNA-Chip gebunden sein. Bevorzugt sind 25-Nukleotid-lange Oligonukleotide. Diese werden bevorzugt aus dem jeweiligen 3'untranslatierten Ende des Gens ausgewählt. Methoden zur Herstellung und Verwendung von DNA-Chips sind z.B. in den US-Patenten Nr. 5,578,832; 5,556,752 und 5,510,270 beschrieben.

Bei cDNA-Chips sind die vollständigen Genprodukte (cDNAs) oder Subfragmente (200-500bp lang) auf dem Chip gebunden. Die Methode wird z. B. in Eckmann L et al., J. Biol. Chem., 2000, 275(19), 14084-14094 beschrieben.

Zuerst werden die geeigneten DNA-Sequenzen gemäß Seq ID No 1 bestimmt. Geeignet sind Sequenzen, die mit den ausgewählten Gentranskripten hybridisieren können. Die Oligonukleotide werden dann durch einen chemischen Prozess, der auf photolithographischen Verfahren basiert, auf dem Chip hergestellt. Dazu werden photolithographische Masken verwendet, die durch geeignete Computer-Algorithmen erzeugt wurden.

Die markierte RNA wird mit dem Chip in einem Hybridisierungsofen inkubiert. Anschließend wird der Chip in einem Scanner analysiert, der die Hybridisierungsprofile bestimmt. Dadurch kann festgestellt werden, ob Veränderungen des Transkripts erfolgt sind (z.B. Mutationen, Trunkierungen). Ebenso ermöglicht es die Quantifizierung des Transkripts und so des HipK4-Proteins und ermöglicht Rückschlüsse auf z.B. eine Mutation im Promoter.

Weiterhin betrifft die Erfindung Verfahren, die auf der Verwendung von RNAi (RNA interference) zur Veränderung des HipK4-Transkriptes beruhen. Eine solche Anwendung kann gezielt zur Schaffung von Kontrazeptiva angewandt werden. Bei der RNAi werden Oligonukleotide basierend auf der Seq ID No 1 eingesetzt, die durch Interaktion mit der körpereigenen HipK4-RNA zur Veränderung des Transkriptes führen.

Die Erfindung betrifft außerdem die Verwendung einer pharmazeutischen Zusammensetzung, die als aktive Komponente eine HipK4-Nukleinsäure enthält, welche in Form eines gentherapeutischen Ansatzes zur Behandlung von männlicher Infertilität verwendet werden kann. Unter Gentherapie versteht man alle Verfahren, die die Ursache genetisch bedingter Erkrankungen durch Veränderung des Genoms behandeln. Bei diesem Verfahren werden die fehlenden Erbinformationen dem Körper direkt zugeführt. Vektoren, die das Gen enthalten, werden injiziert oder inhaliert und gelangen so an ihren Funktionsort.

Die Erfindung betrifft ferner Zellen, die mit einer für den HipK4-Rezeptor kodierenden Nukleinsäuresequenz oder mit einem Vektor transfiziert sind. Als Zellen können z.B. E. coli, Hefe, Pichia, Sf9, HEK-293, CHO, COS, CV-1 oder BHK verwendet werden. Diese Zellen können für die Produktion des HipK4 Polypeptids oder für Testsysteme verwendet werden.

Gegenstand der vorliegenden Erfindung ist auch ein Verfahren zur Herstellung der HipK4-Polypeptide, dadurch gekennzeichnet, dass die Wirtszellen unter Bedingungen kultiviert werden, welche die Expression der DNA-Sequenz erlauben, und wodurch das Expressionsprodukt aus dem Kulturansatz gewonnen werden kann.

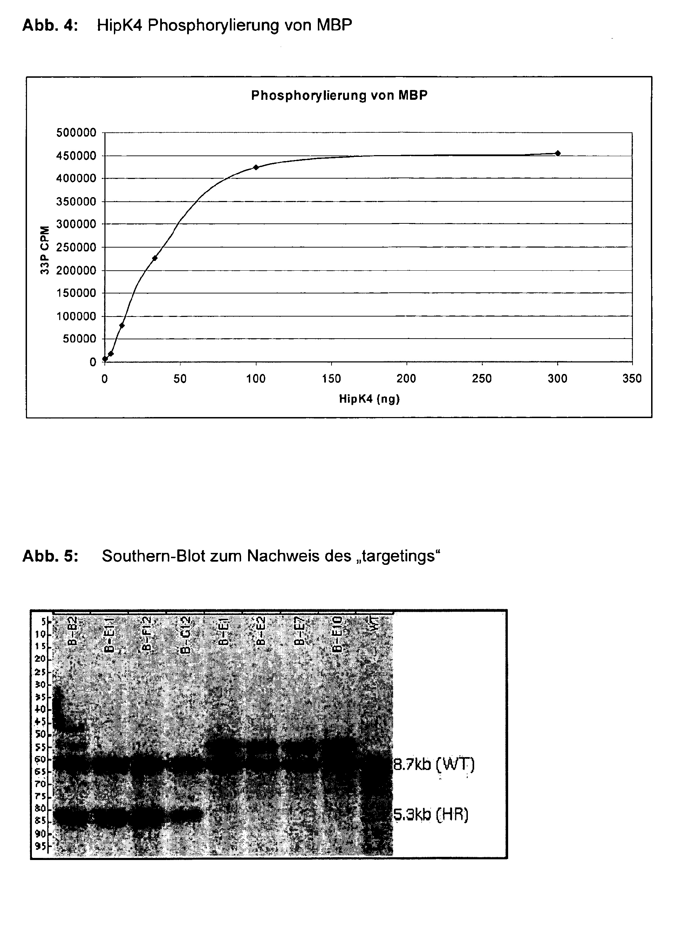

Die Funktion einer Protein-Kinase besteht in der Phosphorylierung von spezifischen Substratproteinen. Dies führt im allgemeinen zu einer Veränderung der biochemischen Eigenschaft des Substrates, was sich durch die Veränderung des Aktivitätszustandes oder der Bindungseigenschaften äußern kann. Die Entwicklung eines Mittels zur Kontrazeption kann nun auf der Regulierung der Aktivität einer Kinase beruhen. Dies setzt jedoch voraus, dass das zu regulierende Protein eine enzymatische Aktivität aufweist und nicht nur als Bindungspartner innerhalb der intrazellulären Kommunikation agiert. Der Nachweis, dass HipK4 eine intrinsische Kinaseaktivität besitzt, erfolgt durch einen Kinase-Assay, bei dem das generelle Kinasesubstrat MBA eingesetzt wird (Abb. 4). Die Intensität der Phosphorylierung erfolgt in Abhängigkeit zur eingesetzten Menge an HipK4. MBA mit seiner Eigenschaft als generelles Kinasesubstrat enthält eine Vielzahl an Serin- und Threonin-Phorphorylierungsresten und ist somit kein ideales Substrat für die Identifizierung von Inhibitoren und der Untersuchung der Kinasekinetik. Ein ideales Substrat wurde durch eine systematische Untersuchung einer Peptidbank identifiziert, wobei die Sequenz der eingesetzten Peptide auf bekannte Phosphorylierungsstellen und Aktivierungsschleifen beschriebener Substrate beruht.

Mit Hilfe eines solchen experimentellen Ansatzes wurden u. a. folgende Proteine als HipK4 Substrate identifiziert; FoxO3a (NP_963853) und RBL2 (NM_005611). Interessanterweise besitzen FoxO3a und RBL2 nachweislich eine wichtige Funktion in der Spermatogenese oder sind essentiell für die Fertilität (Yan, Kero et al. 2001; Hosaka, Biggs et al. 2004). Dass nicht nur HipK4 sondern auch die von HipK4 phosphorylierten Substrate wichtige Bestandteile der Regulierung der Fertilität von Mensch und Maus sind, lässt auf die essentielle Funktion der Kinaseaktivität von HipK4 in den betroffenen Signalwegen schließen. Die Identifizierung von HipK4-Substraten kann jedoch nicht nur zur Klärung der biologischen Funktion beitragen sondern auch Informationen generieren, die zur weiteren Analyse und Entwicklung von HipK4-Modulatoren notwendig sind. Dabei werden die als Substrat identifizierten Peptide zur Etablierung einer auf HipK4 optimierten HTS-Technologie eingesetzt.

Durch Sequenzvergleiche konnte gezeigt werden, dass die HipK4 nur 74 % Sequenzidentität in der ATP-Bindungstasche mit seinen engsten Homologen, der Hipk1 und HipK2 teilt (Tabelle 1). Eine solche geringe Homologie mit anderen Proteinen speziell in der ATP-Bindungstasche, welche die hauptsächliche Zielregion von mutmaßlichen Inhibitoren darstellt, erleichtert die Entwicklung eines spezifischen HipK4-Modulators, weil davon ausgegangen werden kann, dass die HipK4-Modulatoren aufgrund der geringen Sequenzidentität keine weiteren Proteine binden werden.

Ebenfalls Gegenstand der vorliegenden Erfindung ist die Verwendung
a) einer HipK4 Nukleinsäure,
b) eines HipK4 Polypeptids, oder
c) einer Zelle, die HipK4 exprimiert
zur Identifizierung von Effektoren eines HipK4 Polypeptids. Effektoren sind Substanzen, die auf das HipK4 Polypeptid inhibitorisch oder aktivierend wirken, und die in der Lage sind, die HipK4-Funktion der HipK4 Polypeptide zu beeinflussen.

Die Erfindung betrifft weiterhin ein Testsystem zur Identifizierung von Effektoren eines HipK4 Polypeptids, wobei ein HipK4 Polypeptid als ganzes oder Teilsequenzen davon mit einem Modulator inkubiert werden kann und beispielsweise die Bindung eines Moleküls an das HipK4 Polypeptid gemessen wird. Weiterhin betrifft die Erfindung ein Testsystem zur Identifizierung von Effektoren des HipK4 Polypeptids. Hierbei wird aufgereinigtes Enzym mit radioaktiv markiertem ATP und dem Substrat MTB inkubiert und nach einer entsprechenden Inkubationszeit wird der Phosphorylierungsgrad des Substrates bestimmt (Abb. 4).

Ebenfalls Gegenstand der vorliegenden Erfindung ist ein Verfahren zur Bereitstellung eines pharmazeutischen Mittels, wobei
a) Substanzen mit einem Testsystem zur Identifizierung von HipK4 Effektoren in Kontakt gebracht werden,
b) die Wirkung der Substanzen auf das Testsystem im Vergleich zu Kontrollen gemessen wird,
c) eine Substanz, die in Schritt b) eine Modulation der Aktivität des HipK4 Polypeptids zeigt, identifiziert wird,
d) und die in Schritt c) identifizierte Substanz mit in der Pharmazie üblichen Formulierungsstoffen gemischt wird.

Die Effektoren des HipK4 Polypeptids können sowohl zur Diagnose, zur Förderung der Fertilität als auch zur Kontrazeption bei dem Mann eingesetzt werden.

Die bevorzugten Zubereitungen bestehen in einer Darreichungsform, die zur oralen, enteralen oder parenteralen Applikation geeignet ist. Solche Darreichungsformen sind beispielsweise Tabletten, Filmtabletten, Dragees, Pillen, Kapseln, Pulver oder Depotformen sowie Suppositorien. Entsprechende Tabletten können beispielsweise durch Mischen des Wirkstoffs mit bekannten Hilfsstoffen, beispielweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmittel wie Carboxypolymethylen, Carboxymethylcellulose, Celluloseacetatphthalat oder Polyvinylacetat, erhalten werden. Die Tabletten können auch aus mehreren Schichten bestehen.

Ebenfalls kann die erfindungsgemäße pharmazeutische Zusammensetzung zur Diagnose von Infertilität verwendet werden. Hierbei werden die Mengen an HipK4 zum Beispiel durch einen ELISA-Test oder einen Protein-Chip bestimmt.

Gegenstand der vorliegenden Erfindung ist auch die Verwendung der erfindungsgemäßen pharmazeutischen Zusammensetzung zur Diagnose von Autoimmun-Antikörpern in Körperflüssigkeiten.

Die vorliegende Erfindung kann außerdem durch Verwendung der pharmazeutischen Zusammensetzung als Mittel zur Gentherapie Einsatz finden. Hierbei werden die Effekte von HipK4 durch den Einsatz der Gentherapie blockiert. Dabei wird ein Vektor, der eine HipK4-Antisense Sequenz enthält, konstruiert und appliziert. Beispiele sind Vektoren, die aus Adenovirus, Adenovirus-associated virus, Herpes simplex virus oder SV40 abgeleitet sind. Die Gentherapie kann wie beschrieben (Gomez-Navarro et al. 1999, Eur. J. Cancer, 35, 867-885) durchgeführt werden. Die Applikation kann lokal, d.h. direkt in den Bauchraum, oder systemisch, d.h. über den Blutkreislauf, erfolgen. Dies führt zu einer Blockade der Expression von HipK4 im Hoden. Dadurch wird die biologische Funktion, die durch den HipK4 vermittelt wird, gehemmt.

### Biologische Beispiele:

### 1. Klonierung und Expression von humanen HipK4

Der Abschnitt Basenpaar 286-2136 der Seq ID No 1 wird mittels Standard-PCR amplifiziert und über die Restriktionsschnittstellen Hindlll-Notl in den Expressionsvektor pBB4.5 kloniert. Bakuloviren zur Expression von HipK4 in Insektenzellen werden mit Hilfe des "Bac-N-Blue Kits" von Invitrogen nach Anleitung des Herstellers generiert. SF9-Insektenzellen (11) werden bei einer MOl (multiplicity of infection) von 3 mit den HipK4 Bakuloviren infiziert und für 3 Tage kultiviert. Anschließend wird das Zellpellet mit einem Lysispuffer (50 mM Tris, pH 7.5, 150 mM NaCl, Protease Inhibitor Cocktail (Sigma)) aufgeschlossen und das HipK4-Protein mittels Anit-FLAG-Affinitätssäulen (Sigma) nach Gebrauchsanleitung aufgereinigt. Die Effizienz und Reinheit der Aufreinigung wird durch eine Standard SDS-Gel-Elektrophorese mit anschließenden Western-Blot geprüft (Abb. 3).

### 2. RNA-Präparation und Gewebeproben

Zur RNA-Präparation werden die jeweiligen Organe in flüssigen Stickstoff überführt und bis zur weiteren Verarbeitung gelagert (Humane RNA-Proben wurden von Contech erworben). Die RNA-Isolierung erfolgt mittels Trizol (Invitrogen), zur Synthese der cDNA wird das "SuperScript III First Strand" Kit (Invitrogen) verwendet. Beide Verfahren werden strikt nach Anleitung des Herstellers durchgeführt. Hoden und Nebenhoden von Wildtyp und HipK4-defizienten Mäusen werden zur histologischen Analyse präpariert und über Nacht in Bouin's Fixierlösung (0.9 % Pikrinsäure, 9.5 % Formaldehyd und 4.8 % Essigsäure) geschwenkt. Am darauf folgenden Tag werden die Präparate in einer ansteigenden Ethanolreihe dehydriert und in Paraffin eingebettet. Die darauf folgend angefertigten 5 µM Gewebeschnitte können nun mit einer Standard H&E-Färbung behandelt werden.

### 3. Gewebeverteilung in humanen und murinen Gewebe (TaqMan qPCR)

Um die unterschiedlichen Expressionsniveaus von HipK4 in den verschiedenen Geweben in Mensch und Maus zu detektieren, werden Teile der Transkripte mittels PCR quantifiziert (Real-Time TaqMan, Applied Biosystems). Das Haushaltsgen *elongation factor 1 alpha (EF1α)* wird verwendet, um die cDNA Mengen zu normalisieren. Die PCR-Reaktionen werden unter Verwendung des iQ-SYBR Green Supermix (BIO-RAD) entsprechend des Standardprotokolls (Anleitung des Herstellers) angesetzt, wobei folgende Primerpaare verwendet werden (Maus-HipK4 5'-CCGTCTGTCATCCCACAAC-3' und 5'-TAGGGTCCATTCTGGCTCAC-3'; Human-HipK4 5'-CCCGCTGCCCCTTCA-3 und 5'-GCACCTCGCTGAAAATGCT-3'). Um Abweichungen durch Pipettierfehler zu verringern, werden die Reaktionsansätze durch Verwendung eines Mastermixes, der die Primer und den SYBR-Green-Mix enthält, vereinigt. Dieser wird dann auf die vorgelegte cDNA gegeben. PCR-Programm: Denaturierung 95°C, 2 min; Amplifikation (40 Zyklen) 94°C, 15 Sekunden und 60°C, 60 Sekunden. Die DNA-Konzentration wird während der Amplifikation detektiert. Um Expressionsunterschiede zwischen verschiedenen Individuen zu vermindern, werden immer mehrere Gewebeproben zu einem Gesamtpool vereinigt. Um Pipettier- und PCR-Durchführungsfehler zu reduzieren, werden alle PCR-Reaktionen mindestens in Triplikaten angesetzt. Für die spätere Quantifizierung wird der Mittelwert unter Berücksichtigung der Standardabweichung verwendet.

### 4. Gewebeverteilung in Rattengewebe mittels in-situ Hybridisierung

Soweit nicht anders angegeben, werden die Inkubations- und Waschschritte bei Raumtemperatur durchgeführt. Die Paraffinschnitte werden durch zweimaliges Eintauchen in Xylol für jeweils 10 min entwachst und durch eine absteigende Reihe von Ethanol/PBS in reines PBS überführt. Darauf folgt eine Nachfixierung in 4 % PFA für 20 min, gefolgt von zweimaligem Waschen in PBS für jeweils 5 min. Nachdem die Schnitte für 10 min einer Proteinase K-Behandlung unterzogen werden (10 mg/ml Proteinase K in 100 mM Tris-HCl, pH 7,5), werden sie zur Inhibierung der Reaktion für 10 min in 0,2 % Glycerin/PBS eingetaucht. Nach zweimaligem Waschen in PBS für 5 min, wird zum zweiten Mal für 10 min in 4 % PFA fixiert. Dann werden die Schnitte für 5 min mit PBS gewaschen, für 15 min in eine 0,2 N HCI-Lösung eingetaucht, erneut zweimal 5 min mit PBS gewaschen und 10 min in der TEA-Lösung (0,1 M Triethanolamin, pH 8,0; 0,25 % Essiganhydrid) inkubiert, um Aminogruppen abzusättigen. Nun werden die Schnitte für jeweils 5 min in PBS und DEPC-H₂O gewaschen, um sie anschließend für 2 h bei 70°C zu prähybridisieren (in 50 % Formamid; 5 x SSC (20 x SSC: 3 M NaCl; 0,3 M Na-Citrat, pH 7,0), 50 µg/ml Hefe-tRNA; 1 % SDS; 50 µg/ml Heparin; 1 x Denhards; 0,1 % Tween). Die Hybridisierung erfolgt bei 70°C über Nacht mit 3 mg/ml Digoxyginin-markierter Probe in der Prähybridisierungs-Lösung. Am darauf folgenden Tag werden die Schnitte dreimal für 30 min in Lösung 1 (50 % Formamid; 5 x SSC pH 4,5 und 1 % SDS in ddH₂O) bei 67°C gewaschen. Nach drei Inkubationen in TNT (10 mM Tris-HCl, pH 7,5; 0,5 M NaCl; 0.1 % Tween-20) für 5 min bei RT werden die Schnitte zweimal 30 min mit 100 µg/ml RNase in TNT bei 37°C behandelt. Anschließend werden die Schnitte nach einem kurzen Waschschritt für 5 min in TNT: Lösung 11 (1:1) bei RT dreimal für 30 min in Lösung 11 (50 % Formamid; 2 x SSC pH 4,5; 0,2 % SDS, in nicht DEPC-behandeltem Wasser) bei 63°C inkubiert. Nachdem die Schnitte dreimal für jeweils 5 min in MAP-Lösung (100 mM Maleinsäure; 150 mM NaCl; 2 mM Levamisol, pH 7,5) gewaschen werden, folgt das Blockieren der freien Bindungsstellen in MAP/Block/Schafserum (2 % Roche Blockingreagenz in 100 mM Maleinsäure; 150 mM NaCl; 2 mM Levamisol, pH 7,5; 10 % Schafserum) für 3 Stunden bei RT unter konstanter Bewegung auf dem Schüttler. Schließlich werden jeweils 200 ml Antikörper-Lösung (anti-Digoxyginin) pro Objektträger aufgetragen und über Nacht bei 4°C inkubiert. Am dritten Tag der ISH werden die Schnitte dreimal für 10 min und viermal für 1 Stunde in MAP-Lösung gewaschen. Daraufhin werden die Schnitte dreimal 10 min mit NTMT (100 mM Tris-HCl, pH 9,5; 50 mM MgCl2; 100 mM NaCl) gespült. Die Färbereaktion erfolgt anschließend in BM-Purple (Roche, Schweiz), 2 mM Levamisol, so lange, bis das Signal erkennbar ist, maximal über Nacht. Gestoppt wird die Färbung durch dreimaliges Waschen in PBT, pH 4,5, jeweils für 5 min bei RT. Die histologischen Präparate werden zur Aufbewahrung in MOVIOL^{®} eingedeckelt.

### 5. Aktivitätsnachweis (Kinaseassay)

Aufgereinigte Hip-Kinase 4 wird in unterschiedlicher Quantität zusammen mit 20 mM MOPS, pH 7.2, 25 mM β-Glycerolphosphate, 5 mM EGTA, 1 mM Na₃VO₄, 1 mM Dithiothreitol, 10 mM MgOAc, und 25 µM ATP (ink. 10 µCi of [γ-³³P]ATP), zusammen mit 25 µg MBP (Upstate Biotechnology), in einem finalen Volumen von 60 µl inkubiert. Nach einer zweistündigen Inkubation bei Raumtemperatur werden 10 µl des Reaktionsgemisches auf ein Whatman-P81-Phosphozellulosepapier gegeben. Der Filter wird nun mit 0.75 % Phosphorsäure gewaschen, getrocknet und die verbleibende Radioaktivität gemessen. Das Resultat ist in Abb. 3 dargestellt. Zur Identifizierung von putativen Substraten in einem HTS-Verfahren wird das Reaktionsvolumen auf 15 µl reduziert und die Reaktion in einer 384-Loch Mikrotiterplatte durchgeführt. Die Peptide werden in Wasser verdünn und in einer finalen Konzentration von 2.5 µM zum Reaktionsgemisch gegeben. Die Reaktion wird mit 10 ng HipK4 Protein für eine Stunde inkubiert und durch Zugabe von 50 mM EDTA, 3.33 mg/ml Streptavidin-SPA Kügelchen (Amersham) in PBS gestoppt. Die Platte wird für 10 min durchmischt, bei 1000 RPM zentrifugiert und das Scintillations-Signal mittels eines Wallac Mikrobeta-Lesers quantifiziert.

### 6. Generierung eines HipK4 Knockouts

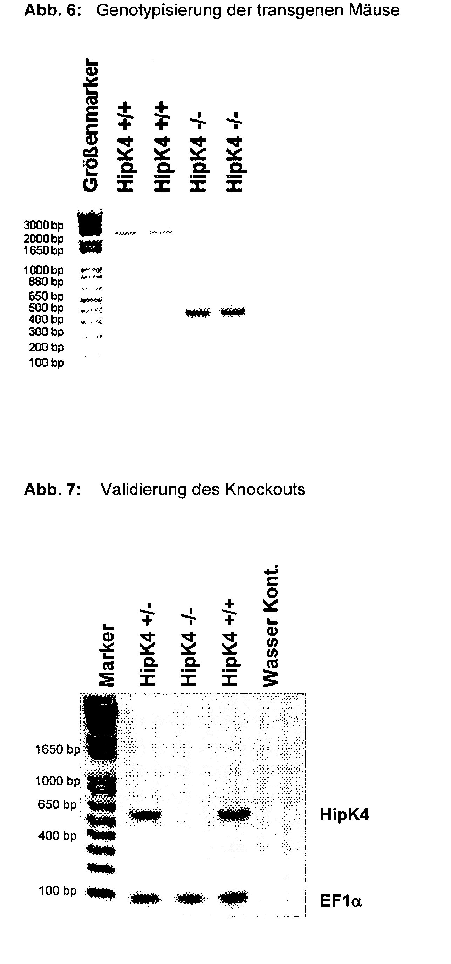

Die Klonierung des Targetingvektors erfolgt mittels einer C57BL/6J BAC-DNA-Sammlung (Artemis). Erfolgreiche homologe Rekombination führt zur Deletion von Exon 2-3 des murinen HipK4-Allels und somit auch zu einer partiellen Deletion der Proteinkinase-Domäne. Der ES-Zellklon B-B2 (genetischer Hintergrund C57BU6 N) wird mittels Southern-Blot als positiv bezüglich der homologen Rekombination identifiziert (Abb. 5) (Artemis). Hierzu wird die genomische DNA von diversen ES-Zellklonen mit dem Restriktionsenzym Hind III verdaut und über ein Agarosegel aufgetrennt. Nachdem die verdaute DNA per Southern-Blot auf eine Nitrozellulosemembran transferiert wird, können die durch die homologe Rekombination entstandenen Restrikitons-Fragment-Längen-Polymorphismen (RFLPs) durch Verwendung einer 3'externen Sonde detektiert werden (Abb. 5). Zu erwarten sind zwei Fragmente für Wildtypklone und 3 Fragmente für Klone mit erfolgreicher homologen Rekombination auf einem Allel. 11,2 kb und 8,7 kb große Fragmente für den Wildtypklon und ein zusätzliches 5,3 kb großes Fragment für Klone mit erfolgreicher homologer Rekombination (HR). Der ES-Zellklon B-B-2 wird zur Injektion in Blastozyten C57BU6 N verwendet und die daraus resultierenden Chimären mit C57BU6 N-Weibchen verpaart. Das Genotypisieren der transgenen Nachkommen wird mittels PCR-Analyse mit Einsatz von genomischer DNA aus der Schwanzspitze durchgeführt. Die hierzu eingesetzten Primer HipK4-A 5'CTACTGCACAACTAAATCTGTAGC-3' und HipK4-B 5'-CAGCGGTAGAGGAAGATAGAGG-3' hybridisieren in Intron 2 (HipK4-A) und in Intron 3 (HipK4-B) des murinen HipK4-Gens. Bei der Verwendung dieser Primer wird für das Wildtypallel ein 364 Basenpaar und für das Knockoutallel ein 2850 Basenpaar großes Fragment vermehrt (Abb. 6). Die Generierung von homozygoten Knockouts erfolgt durch Verpaarung von heterozygoten Mäusen. Der Nachweis des Fehlens eines HipK4-Transkriptes in den homozygoten Knockout-Tieren kann mit einer klassischen RT-PCR erbracht werden (Abb. 7). Hierzu werden folgende Primer eingesetzt: HipK4-C 5'-TGCAGACTCAGGTCATCGAG-3' und HipK4-D 5'-GCAAGGCTCACCACTTCTTC-3' sowie die folgenden Primer für die Kontrollreaktion mit dem *elongation factor 1 alpha:* E-A 5'AATTCACCAACACCAGCAGCAA-3' E-B 5'-TGCCCCAGGACACAGAGACTTCA-3').

### Abbildungen:

Abb.1: Expression in verschiedenen Geweben (TaqMan qPCR), Vergleich Mensch (Abb 1 B) / Maus (Abb 1A).
In Mäusen sowie im Menschen wird die HipK4 vor allem im Hoden exprimiert (Abb 1 A und B). In der Maus konnte außerdem eine Expression im Gehirn nachgewiesen werden, die 37 % der Intensität des Expressionsniveaus des Hodens aufweist (Abb 1 B).

Abb. 2: Expression von HipK4 im Rattenhoden (in-situ Hybridisierung)
HipK4 wird exklusiv in postmeiotischen runden Spermatiden exprimiert. In Kombination mit einer PNA-Lektin und Propidiumjodid-Färbung konnte die Expression von HipK4 während der Spermiogenese auf die Entwicklungsstufe 4-6 eingeschränkt werden.

Abb. 3: Expression und Aufreinigung von HipK4
Standard SDS-Gel-Elektrophorese zur Überprüfung der Effizienz und Reinheit der Aufreinigung von humaner HipK4.

Abb. 4: HipK4 Phosphorylierung von MBP
Das generelle Kinasesubstrat MBA wird phosphoryliert. Die Intensität der Phosphorylierung erfolgt in Abhängigkeit zur eingesetzten Menge an HipK4.

Abb. 5: Southern-Blot zum Nachweis des "targetings"
Positive Identifizierung des ES-Zellklons B-B2 mittels Southern-Blot in bezug auf die homologe Rekombination.

Abb. 6: Genotypisierung der transgenen Mäuse
Transgenen Nachkommen wird zur Genotypisierung aus der Schwanzspitze genomische DNA entnommen, die mittels PCR analysiert wird.

Abb. 7: Validierung des Knockouts
Nachweis des Fehlens des HipK4 Transkripts in homozygoten Knockout-Tieren mittels RT-PCR

Abb. 8 und Abb. 9: Vergleich von Hoden von Wildtyp- mit Knockout-Tieren
Histologische Analyse von Hoden und Nebenhoden. Im Hoden führt der Mangel an HipK4 zu einer Beeinflussung der Spermiogenese (untypische Anordnung der sich entwickelnden Spermatiden, Abb. 8, Pfeil). Der Spermiogenesedefekt führt zur Freisetzung einer verminderten Anzahl an Spermien im Hoden (Abb 9).

### Tabelle 1: Identität zwischen HipK4 und anderen Proteinen

Die HipK4 teilt nur 74 % Sequenzidentität in der ATP-Bindungstasche mit seinen engsten Homologen, der HipK1 und HipK2.

## Patentansprüche

1. Verwendung einer pharmazeutischen Zusammensetzung, die als aktive Komponente mindestens eine Substanz, ausgewählt aus der Gruppe von einer HipK4-Nukleinsäure entsprechend Seq ID No 1, einem HipK4-Polypeptid, entsprechend Seq ID No 2, Modulatoren der HipK4, Antikörpern, die gegen die HipK4 gerichtet sind, einer HipK4-Antisense sowie einer HipK4-RNAi, zur Diagnose und Behandlung von Fertilitätsstörungen, zur Kontrazeption und auch zur Herstellung eines Medikaments für die Behandlung von Fertilitätsstörungen.

2. Verwendung der pharmazeutischen Zusammensetzung nach Anspruch 1, wobei die Nukleinsäure einen Protein-kodierenden Abschnitt, der der in Seq ID No 1 dargestellten Nukleinsäuresequenz entspricht, umfasst.

3. Verwendung der pharmazeutischen Zusammensetzung nach Anspruch 1, wobei die Nukleinsäure für ein Polypeptid mit der in Seq ID No 2 dargestellten Aminosäuresequenz kodiert.

4. Verwendung gemäß Anspruch 3, wobei der proteinkodierende Abschnitt im Bereich von Nukleotid 286 - 2136 der in Seq ID No 1 dargestellten Sequenz liegt.

5. Verwendung der pharmazeutischen Zusammensetzung nach Anspruch 1, wobei das enthaltene HipK4 Polypeptid ein Polypeptidfragment umfasst, das im Bereich von Position 1 bis Position 616 der Aminosäuresequenz gemäß Seq ID No 2 liegt.

6. Verwendung der pharmazeutischen Zusammensetzung nach Anspruch 1, wobei die enthaltenen Antikörper gegen das HipK4-Polypeptid gerichtet sind, zur Diagnose von Fertilitätsstörungen.

7. Verwendung der pharmazeutischen Zusammensetzung nach Anspruch 1, wobei die Zusammensetzung eine Nukleinsäuresonde enthält, die komplementär zu den Nukleinsäuresequenzen sind, die für das Polypeptid, das die in Seq ID No 4 dargestellte Aminosäuresequenz umfasst, kodiert, zur Diagnose von Fertilitätsstörungen.

8. Verwendung gemäß Anspruch 7, wobei die Nukleotidsequenzen mit einem nachweisbaren Marker versehen sind.

9. Verwendung der pharmazeutischen Zusammensetzung nach Anspruch 1, wobei die Zusammensetzung Moleküle enthält, die die Expression von HipK4 unterdrücken können, zur Kontrazeption.

10. Verwendung gemäß Anspruch 9, wobei es sich bei dem Molekül um ein Antisense-Molekül handelt.

11. Verwendung der pharmazeutischen Zusammensetzung nach Anspruch 1, die als aktive Komponente mindestens eine HipK4 Nukleinsäure, oder ein HipK4 Polypeptid oder ein HipK4 Antisense-Molekül als Zielsubstanz zur Herstellung eines Mittels für die Behandlung von Krankheiten, die ursächlich mit dem HipK4 Gen und/oder Protein zusammenhängen, enthält.

12. Verwendung der pharmazeutischen Zusammensetzung nach Anspruch 1 als Mittel zur Gentherapie, wobei die aktive Komponente eine HipK4-Nukleinsäure ist.

13. Verfahren zur Herstellung der Proteine oder Polypeptide, die in der pharmazeutischen Zusammensetzung gemäß Anspruch 4 und 5 enthalten sind, **dadurch gekennzeichnet, dass** Wirtszellen unter Bedingungen kultiviert werden, welche die Expression der DNA-Sequenz erlauben und wodurch das Expressionsprodukt aus dem Kulturansatz gewonnen werden kann.

14. Verwendung der Proteine oder Polypeptide, die in der pharmazeutischen Zusammensetzung gemäß Anspruch 1 enthalten sind, zur Isolierung und Herstellung von spezifischen Liganden.

15. Verwendung einer pharmazeutischen Zusammensetzung nach Anspruch 1, die eine HipK4 Nukleinsäure, ein HipK4 Polypeptid oder eine Zelle, die HipK4 exprimiert, enthält, zur Identifizierung von Effektoren eines HipK4 Polypeptids.

16. Testsystem zur Identifizierung von Effektoren eines HipK4 Polypeptids, wobei ein HipK4 Polypeptid als ganzes oder Teilsequenzen davon mit einem Modulator inkubiert wird und die Bindung eines Moleküls an das HipK4 Polypeptid gemessen wird, wobei HipK4 mit radioaktiv markiertem ATP und dem Substrat MBP inkubiert wird und nach einer entsprechenden Inkubationszeit der Phosphorylierungsgrad des Substrates bestimmt wird.

17. Verwendung der pharmazeutischen Zusammensetzung nach Anspruch 1, wobei es sich bei dem Effektor um einen Inhibitor der HipK4 handelt, zur Kontrazeption.

18. Verfahren zur Bereitstellung eines pharmazeutischen Mittels, wobei
a) Substanzen mit einem Testsystem nach Anspruch 16 zur Identifizierung von HipK4 Effektoren in Kontakt gebracht werden,
b) die Wirkung der Substanzen auf das Testsystem im Vergleich zu Kontrollen gemessen wird,
c) eine Substanz, die in Schritt b) eine Modulation der Aktivität des HipK4 Polypeptids zeigt, identifiziert wird,
d) und die in Schritt c) identifizierte Substanz mit in der Pharmazie üblichen Formulierungsstoffen gemischt wird.

19. Verwendung eines erfindungsgemäßen DNA-Chips zur Diagnose von Fertilitätsstörungen vorzugsweise im Hoden und Ovidukt.

20. Verwendung der pharmazeutischen Zusammensetzung gemäß Anspruch 1, wobei eine siRNA enthalten ist, die die Veränderung des HipK4-Transkriptes zur Folge hat, zur Kontrazeption.
